(19) Europäisches Patentamt
European Patent Office
Office européen des brevets

(11) Veröffentlichungsnummer: **0 468 481 A1**

(12) **EUROPÄISCHE PATENTANMELDUNG**

(21) Anmeldenummer: **91112428.7**

(22) Anmeldetag: **24.07.91**

(51) Int. Cl.$^5$: **G01N 33/53**, G01N 33/543, C08L 71/02, //G01N33/74

(30) Priorität: **25.07.90 DE 4023671**

(43) Veröffentlichungstag der Anmeldung: **29.01.92 Patentblatt 92/05**

(84) Benannte Vertragsstaaten: **AT BE CH DE DK ES FR GB GR IT LI LU NL SE**

(71) Anmelder: **BOEHRINGER MANNHEIM GMBH**
**Sandhofer Strasse 112-132**
**W-6800 Mannheim-Waldhof(DE)**

(72) Erfinder: **Sluka, Peter, Dr.**
**Rosenstrasse 39**
**W-8120 Weilheim(DE)**
Erfinder: **Klein, Christian, Dr.**
**Blütenstrasse 16**
**W-8120 Weilheim(DE)**
Erfinder: **Griesser, Hans-Werner, Dr.**
**Von-Kühlmann-Strasse 11 A**
**W-8132 Tutzing(DE)**
Erfinder: **Kobold, Uwe, Dr.**
**Veilchenweg 7**
**W-8120 Weilheim(DE)**

(74) Vertreter: **Huber, Bernhard, Dipl.-Chem. et al**
**Patentanwälte H. Weickmann, Dr. K. Fincke**
**F.A. Weickmann, B. Huber Dr. H. Liska, Dr. J.**
**Prechtel Kopernikusstrasse 9 Postfach 86 08**
**20**
**W-8000 München 86(DE)**

(54) **Nichtionische Blockcopolymere aus Propylenoxid und Ethylenoxid.**

(57) Die Erfindung betrifft ein Verfahren zur Bestimmung eines Partners in einer Immunreaktion nach dem Immunoassay-Prinzip, wobei einer der Reaktionspartner in fester Phase vorliegt, wobei man als oberflächenaktives Mittel ein Polyethylenoxid-Polypropylenoxid-Blockcopolymer der Formel I

$$HO(CH_2CH_2O)_a(\overset{CH_3}{\overset{|}{C}}H-CH_2O)_b(CH_2CH_2O)_aH$$

einsetzt und worin a einen Wert zwischen 40 und 150 und b einen Wert zwischen 10 und 50 annehmen kann, worin man eine hydrophile Blockcopolymer-Zusammensetzung der Formel I verwendet, die in 1%iger wäßriger Lösung (Gew./Vol) eine Oberflächenspannung von mindestens 45 mN/m aufweist und bei der das mittlere molare Verhältnis von Ethylenoxid- zu Propylenoxidgruppen (2a/b) in der Zusammensetzung mindestens 5,8 beträgt. Weiterhin beinhaltet die Erfindung ein Reagenz zur Durchführung des obigen Verfahrens.

EP 0 468 481 A1

Nichtionische Blockcopolymere aus Propylenoxid und Ethylenoxid, sogenannte Poloxamere, sind bereits seit längerem bekannt und haben als schaumarme Tenside eine breite Anwendung z.B. als schwach schäumende Komponenten in Reinigungsmitteln, Solubilisatoren, Verdickern, Emulgatoren und Dispergiermitteln gefunden (siehe z.B. Ullmanns Enzyklopädie der technischen Chemie, Band 19, 4. Auflage, Verlag Chemie, Weinheim).

Der Einsatz dieser Blockcopolymere als oberflächenaktive Mittel für Immunoassays in heterogener Phase wurde in der EP-A 0 215 457 offenbart.

Bei einem Immunoassay in heterogener Phase liegt einer der Reaktionspartner, vorzugsweise ein Antikörper, an einen Träger gebunden vor. Zur Durchführung der Immunbestimmung in heterogener Phase sind verschiedene Verfahrensarten bekannt, z.B. das Sandwich-Verfahren, das indirekte Verfahren, das Kompetitionsverfahren und weitere Verfahren, die in der EP-A 0 215 457 näher beschrieben sind.

Bei Immunbestimmungen in heterogener Phase treten häufig unspezifische Störungen auf, die verschiedene Ursachen haben und unter anderem als "Matrixeffekt", "Background" und "unspezifische Bindung" bezeichnet werden. Dabei stellt man insbesondere bei Plasmaproben eine schlechte Wiederfindung der zu bestimmenden Substanz fest.

Gemäß der EP-A-0 215 457 wird nun zur Verringerung der unspezifischen Bindung ein Verfahren zur Bestimmung eines Partners einer Immunreaktion nach dem Immunoassay-Prinzip bei einer Temperatur zwischen 15 und 40 °C vorgeschlagen, wobei einer der Reaktionspartner in fester Phase vorliegt, welches dadurch gekennzeichnet ist, daß man ein oberflächenaktives Mittel mit einem HLB-Wert von mehr als 20 zusetzt. Als oberflächenaktive Mittel sind nichtionische Blockcopolymere auf Basis von Alkylenoxiden mit 2 bis 4 C-Atomen, insbesondere Verbindungen der Formeln I

$$HO(CH_2CH_2O)_a(\underset{CH_3}{\overset{|}{C}}H\text{-}CH_2O)_b(CH_2CH_2O)_aH$$

verwendet, worin a einen Wert zwischen 30 und 150 und b einen Wert zwischen 15 und 60 annehmen kann. Das molare Verhältnis von Polyethylenoxid- zu Polypropylenoxidgruppen (2a/b) kann also von 1 bis 20 betragen.

Die oberflächenaktive Verbindung gemäß Formel I kann dabei unspezifische Wechselwirkungen bestimmter Bestandteile der Probe (Plasma) mit der Oberfläche des Reaktionsgefäßes verhindern und führt gleichzeitig nicht zur Ablösung des heterogenen, d.h. an die Oberfläche adsorbierten Reaktionspartners.

Für heterogene immunologische Testsysteme gemäß EP-A-0 215 457 werden bestimmte Typen der kommerziell erhältlichen Poloxamere der Pluronic- bzw. Synperonic-Reihe (Hersteller: BASF bzw. ICI) verwendet, deren Spezifikation den geforderten Eigenschaften entspricht. Überraschenderweise zeigte sich jedoch, daß bei einigen Tests die Wiederfindung des Analyten sowie die Steilheit der Eichkurve eine große Abhängigkeit von der jeweils eingesetzten Herstellercharge des kommerziellen Tensids zeigte. Es wurde festgestellt, daß für diese Tests nur sehr wenige ausgewählte Chargen eingesetzt werden können. Daher ist es erforderlich, daß die Eignung der einzelnen Chargen jeweils mit einem aufwendigen Funktionstest ermittelt werden muß.

Aufgabe der vorliegenden Erfindung war es somit, eine zur Verwendung in einem heterogenen Immunoassay reproduzierbar geeignete Blockcopolymer-Zusammensetzung der Formel I bereitzustellen.

Die erfindungsgemäße Aufgabe wird durch ein Verfahren zur Bestimmung eines Partners in einer Immunreaktion nach dem Immunoassay-Prinzip gelöst, wobei einer der Reaktionspartner in fester Phase vorliegt, und wobei man als oberflächenaktives Mittel ein Polyethylenoxid-Polypropylenoxid-Blockcopolymer der Formel I

$$HO(CH_2CH_2O)_a(\underset{CH_3}{\overset{|}{C}}H\text{-}CH_2O)_b(CH_2CH_2O)_aH$$

einsetzt und worin a einen Wert zwischen 40 und 150 und b einen Wert zwischen 10 und 50 annehmen kann, welches dadurch gekennzeichnet ist, daß man eine hydrophile Blockcopolymer-Zusammensetzung der Formel I verwendet, die in 1%iger wäßriger Lösung (Gew./Vol.) eine Oberflächenspannung von

mindestens 45 mN/m aufweist und bei der das mittlere molare Verhältnis von Polyethylenoxid- zu Polypropylenoxidgruppen (2a/b) in der Zusammensetzung mindestens 5,8 beträgt.

Üblicherweise liegen die Blockpolymeren nicht als chemische Einzelverbindungen vor, sondern als Gemisch aus mehreren Einzelverbindungen mit unterschiedlichem Molekulargewicht und unterschiedlichem molaren Verhältnis von Polyethylenoxid(EO)zu Polypropylenoxid(PO)gruppen vor. Aus diesem Grund sind die Werte a und b als Durchschnittswerte zu verstehen, die für das Gemisch aus dem Molekulargewicht und dem molaren Verhältnis (EO/PO) ermittelt werden können.

Bei dem erfindungsgemäßen Verfahren hat es sich als günstig erwiesen, die oberflächenaktive Substanz, d.h. die hydrophile Blockcoplymer-Zusammensetzung der Formel I in einer Menge von 0,1 bis 5 %, bezogen auf das Gewicht des gesamten Reaktionsansatzes, einzusetzen.

Die Verwendung einer hydrophilen Blockcopolymer-Zusammensetzung gemäß vorliegender Erfindung bringt sowohl Vorteile bei der Bestimmung von Substanzen im Plasma als auch in Serum. In beiden Fällen tritt eine Beschleunigung der Reaktion sowie eine verbesserte Wiederfindung der zu bestimmenden Substanz, insbesondere bei Bestimmungen im Plasma gegenüber der Verwendung eines üblichen kommerziellen Blockcopolymers auf.

Das erfindungsgemäße Verfahren ist auf alle Plasmaarten anwendbar. So kann man Substanzen auch in Plasma bestimmen, das zur Stabilisierung mit EDTA, Heparin oder Citrat versetzt wurde.

Der Zusatz der hydrophilen Blockcopolymer-Zusammensetzung gemäß vorliegender Erfindung kann bei jeder der für die Bestimmung verwendeten Lösungen erfolgen. Führt man z.B. eine Bestimmung nach dem Sandwichprinzip durch, so wird in einem ersten Schritt ein Antikörper an ein Trägermaterial adsorbiert, wobei nach diesem Adsorptionsschritt zweckmäßigerweise eine Behandlung mit einer Rinderserumalbuminlösung (zur Absättigung der Oberfläche) und anschließendes Waschen mit einer Salz/Detergenslösung erfolgt. Im nächsten Schritt wird die Testlösung zugegeben, welche die zu bestimmende Substanz und einen markierten spezifischen Antikörper gegen den Komplex aus Antigen und gebundenem ersten Antikörper oder gegen Teile dieses Komplexes enthält. Vorzugsweise ist dabei der markierte Antikörper mit einem Enzym, z.B. Peroxidase, konjugiert. Über den an die Festphase gebundenen markierten Antikörper kann dann die Menge des Antigens, d.h. der zu bestimmenden Substanz, berechnet werden. Bei einem erfindungsgemäßen Verfahren befindet sich das hydrophile Blockcopolymer in der Testlösung.

Das mittlere molare Verhältnis von Ethylenoxid- zu Propylenoxidgruppen in einer hydrophilen Blockcopolymer-Zusammensetzung, die für ein erfindungsgemäßes Verfahren geeignet ist, beträgt mehr als 5,8, d.h. die Blockcopolymer-Zusammensetzung enthält mindestens 81,5 Gew.-% Ethylenoxid. Der Begriff "Zusammensetzung" im Sinne der Erfindung bedeutet, daß das Blockcopolymer, wie bereits oben ausgeführt, nicht homogen sein muß, sondern in der Regel aus mehreren Komponenten bzw. Fraktionen mit unterschiedlichem Molekulargewicht bzw. unterschiedlichem molarem Verhältnis von Ethylenoxid- zu Propylenoxidgruppen zusammengesetzt ist. Das mittlere molare Verhältnis von Ethylenoxid- (EO) zu Propylenoxid-(PO)-Gruppen in der Zusammensetzung beträgt mindestens 5,8. Das bedeutet, daß in der erfindungsgemäßen Zusammensetzung auch Komponenten bzw. Fraktionen des Blockcopolymers vorliegen können, bei denen das Verhältnis von EO- zu PO-Gruppen weniger als 5,8 beträgt, solange das molare Verhältnis in der Gesamtzusammensetzung mindestens 5,8 beträgt. Der Anteil von Fraktionen mit EO/PO <5 sollte jedoch nicht mehr als 25 Gew.-%, vorzugsweise nicht mehr als 5 Gew.-% der Zusammensetzung betragen.

Bevorzugt ist die Verwendung einer hydrophilen Blockcopolymer-Zusammensetzung der Formel I, die in 1%iger wäßriger Lösung (Gew./Vol.) eine Oberflächenspannung von 45 bis 55 mN/m aufweist und bei der das mittlere molare Verhältnis der Ethylenoxid- zu Propylenoxidgruppen (2a/b) 5,8 bis 15 beträgt. Vorzugsweise ist b größer als 15 und besonders bevorzugt nicht größer als 22. Vorzugsweise liegt a zwischen 50 und 70, besonders bevorzugt zwischen 53 und 63.

Eine für ein erfindungsgemäßes Verfahren geeignete hydrophile Blockcopolymer-Zusammensetzung kann man auf einfache Weise durch Fraktionierung eines kommerziellen Blockcopolymers gewinnen, bei dem es sich um eine Verbindung nach der allgemeinen Formel I handelt und das in seiner Gesamtheit nicht als oberflächenaktives Mittel in einem Immunoassay geeignet ist.

Überraschenderweise wurde gefunden, daß bei Chromatographie von ungeeigneten Chargen eines kommerziellen Blockcopolymers der Formel I über eine Diaion HP20 Säule und Elution mit einem Wasser/Isopropanol-Gradienten hydrophile Fraktionen des kommerziellen Blockcopolymers gewonnen werden konnten, welche einzeln für die kritischen Tests voll einsetzbar sind. Es konnten auch Gemische aus verschiedenen geeigneten Fraktionen eingesetzt werden. Beispiele für geeignete kommerzielle Blockcopolymere, aus denen geeignete Fraktionen oder "Zusammensetzungen" isoliert werden können, sind etwa Synperonic® PE/F-68 (ICI), Pluronic® F-68 (BASF), Genapol® PF80 (Hoechst) und Texadril® 8780 (Henkel).

Bei einem erfindungsgemäßen Verfahren ist es bevorzugt, daß man eine hydrophile Blockcopolymer-Zusammensetzung verwendet, die im wesentlichen frei von hydrophoben Anteilen ist, bei denen das molare Verhältnis von Ethylenoxid zu Propylenoxidgruppen (2a/b) weniger als 5 beträgt. Unter im wesentlichen frei versteht man, daß ein hydrophiles Blockpolymer weniger als 1 % und bevorzugt weniger als 0,5 % hydrophobe Anteile enthält, die durch einen hohen Anteil an Propylenoxidgruppen gekennzeichnet sind.

Besonders geeignet hat sich das erfindungsgemäße Verfahren zur Bestimmung von luteinisierendem Hormon (LH) oder Follikel stimulierendem Hormon (FSH) erwiesen.

Weiterhin ein Gegenstand der vorliegenden Erfindung ist ein Reagenz zur Durchführung eines erfindungsgemäßen Verfahrens, das als oberflächenaktives Mittel ein Polyethylenoxid-Polypropylenoxid-Blockcopolymer der allgemeinen Formel I

$$HO(CH_2CH_2O)_a(\overset{\displaystyle CH_3}{\overset{|}{C}}H-CH_2O)_b(CH_2CH_2O)_aH$$

enthält, worin die Blockcopolymer-Zusammensetzung in einer 1 %igen wäßrigen Lösung (Gew./Vol.) eine Oberflächenspannung von mindestens 45 mN/m aufweist und das mittlere molare Verhältnis von Ethylenoxid- zu Propylenoxidgruppen (2a/b) in der Zusammensetzung mindestens 5,8 beträgt. Vorzugsweise enthält das erfindungsgemäße Reagenz eine oder mehrere geeignete Fraktionen eines kommerziellen Polyethylenoxid-Polypropylenoxid-Blockcopolymers der allgemeinen Formel I.

Zur Durchführung des erfindungsgemäßen Verfahrens wird ein Reagenz verwendet, welches mobile oder/und immobilisierte Partner der Immunreaktion und außerdem noch an sich übliche Inhaltsstoffe enthalten kann. Zweckmäßigerweise enthält es auch Puffersubstanzen, z.B. Phosphatpuffer, Citratpuffer, Boratpuffer und dergleichen oder/und Rinderserumalbumin oder/und Konservierungsmittel. Wird als Markierung bei der Immunreaktion ein Enzym verwendet, so enthält das Reagenz noch ein System zum Nachweis der Enzymaktivität.

Mit dem erfindungsgemäßen Verfahren und Reagenz gelingt es, die Wiederfindung bei Immunoassays in heterogener Phase gegenüber einem Verfahren, bei dem man ein kommerziell erhältliches Blockpolymer der Formel I verwendet, zu verbessern.

Die nachfolgenden Beispiele und die Abbildung erläutern die Erfindung weiter:

Fig. 1 zeigt Eichkurven des LH-Tests mit unfraktioniertem Tensid (1), Referenzcharge (2) und fraktoniertem Tensid, Fraktion 5 (s. Tabelle 1) (3).

## Beispiele

Beispiel 1

Tensidfranktionierung

Das Tensid Synperonic® PE/F-68 (Hersteller: ICI, Polypropylenoxid-Polyethylenoxid-Blockcopolymer) war in der vom Hersteller gelieferten Form für den Einsatz im Enzymun-Test zum Nachweis von luteinisierendem Hormon (LH) und Follikel stimulierendem Hormon (FSH) nicht geeignet. Eine 40%-ige (w/v) wässrige Lösung des Tensids wurde auf eine Säule mit dem 5-fachen Volumen an Diaion HP 20 (Hersteller: Mitshubishi Chemicals) aufgetragen und anschließend mit einem gestuften Wasser/Isopropanol-Gradienten mit steigendem Isopropanolanteil eluiert. Von jeder Gradientenstufe wurden 3 Fraktionen aufgefangen und das Lösungsmittel abgedampft. Die geeigneten Fraktionen wurden bei einem Isopropanolgehalt im Lösungsmittelgemisch zwischen 20 und 40% eluiert. Es wurden insgesamt 12 Fraktionen erhalten, die anschließend charakterisiert und im Funktionstest nach Beispiel 2 bewertet wurden. Die analytischen Daten der Fraktionen sind in Tabelle I dargestellt.

Zum Vergleich sind zunächst die analytischen Daten einer Referenzcharge eines für immunologische Bestimmungen (z.B. für die Bestimmung von LH und FSH) geeigneten Block-Copolymers der allgemeinen Formel I angegeben:

| EO/PO | OF-Span. | $MM_n$ | b | a |
|---|---|---|---|---|
| 6,21 | 45,2 | 5700 | 17 | 53 |

Tabelle I

Analytische Daten zur Fraktionierung der ungeeigneten Charge 13 von Synperonic PE/F-68:

| Fraktion | EO/PO | OF-Span. (1% Lsg.) | $MM_n$ | b | a | Gew.-Anteil ( % ) |
|---|---|---|---|---|---|---|
| unfraktioniert | 5.65 | 40.0 | 5800 | 19 | 54 | 100 |
| 1 | 14.43 | 51.8 | | | | 0.8 |
| 2 | - | - | | | | 0.3 |
| 3 | 13.13 | 49.6 | | | | 0.8 |
| 4 | 9.08 | 49.0 | 6500 | 14 | 63 | 11.7 |
| 5 | 7.64 | 48.3 | 5500 | 14 | 53 | 8.8 |
| 6 | 5.82 | 45.2 | 6800 | 22 | 63 | 25.8 |
| 7 | 4.47 | 43.8 | 6800 | 27 | 60 | 22.0 |
| 8 | 4.13 | 38.4 | 6900 | 29 | 59 | 10.1 |
| 9 | 3.65 | 41.2 | 7060 | 32 | 59 | 10.8 |
| 10 | 3.53 | 40.4 | | | | 2.7 |
| 11 | 3.30 | 39.7 | 6500 | 32 | 53 | 5.4 |
| 12 | 2.87 | 38.1 | | | | 0.5 |

EO/PO:

Molares Verhältnis von Ethylenoxid zu Propylenoxidgruppen. Wird berechnet aus den Signalintensiitäten der Oxymethylen- und Methylprotonen im $^1$H-NMR nach der allgemeinen Formel:

$$EO/PO = \frac{I_o - I_M}{I_M} \cdot 0,75$$

$I_o$ = integrale Signalintensität der Oxymethylenprotonen

$I_M$ = " " " Methylprotonen

Die $^1$H-NMR-Spektren wurden in $D_2O$ mit einem Bruker 100 MHz-Spektrometer aufgenommen.

OF-Span. (Oberflächenspannung):

Wurde in 1 %iger (w/v) Lösung des Polymers in bidest. Wasser nach der Methode von Wilhelmy bestimmt (C. Weser; GIT Fachzeitschrift für das Laboratorium, 24, 642 - 648 und 734 - 742 (1980)). Verwendet wurde ein Digital-Tensiometer der Fa. Krüss.

$MM_n$:

Zahlenmittel der Molmasse. Wurde mittels Endgruppenanalyse bestimmt (C. Ogg, W.L. Porter, C.O. Willits, Ind. Eng. Chem., Anal Ed. 17, 394 - 7 (1945))

Gew.-Anteil:

Anteil der Fraktionen am Gesamtpolymer:

$$\frac{\text{Gewicht der Fraktion (g)}}{\text{Gewicht des eingesetzten Polymers (g)}} \cdot 100$$

Die Fraktionen 4, 5, 6 und ein Pool der Fraktionen 4 - 6 wurden getestet. Die Fraktionen 4, 5 und der Pool waren voll tauglich bezogen auf Eichkurve und Wiederfindung (bei einer max. zulässigen Abweichung zur Referenzcharge von 10 %). Fraktion 6 ist nur bedingt tauglich (Eichkurve zu flach), war aber im Pool voll tauglich.

Beispiel 2

Bestimmung des luteinisierenden Hormons (LH) nach dem Sandwich-Prinzip
1,5 ml einer Lösung, die einen monoklonalen Antikörper gegen LH (EP-A 0 193 881) in Beschichtungspuffer (0,04 mol/l Natriumdihydrogenphosphat, pH 7,4) in einer Konzentration von 1,5 µg/ml enthält, werden in Plastikröhrchen gegeben und 24 Stunden bei Raumtemperatur inkubiert. Anschließend wird mit einer Lösung von 1 % Rinderserumalbumin in 0,9 % NaCl 30 min bei Raumtemperatur inkubiert. Nach Waschen und Trocknen werden in jedes Röhrchen 100 µl Probe (Serum oder LH-Standardlösung mit 0 - 25 mU LH/ml, standardisiert nach dem ersten IRP-Standard für LH Code 68/40, erster IRP = erste internationale Referenzpräparation der WHO) zusammen mit 1 ml einer Arbeitslösung gegeben und 120 min inkubiert. Diese Arbeitslösung enthält ein Anti-LH-Peroxidasekonjugat (80 mU/ml Konjugat aus Antikörper nach EP-A-0 193 881 und Peroxidase) in 0,04 mol/l Phosphatpuffer pH 7,4, Blockpolymer nach Beispiel 1 (6 g/l) und PEG 40000 (10 g/l).

Nach Waschen mit Waschpuffer (0,9 % NaCl, 0,1 % Tween 20) wird eine Lösung aus 100 mmol/l Phosphat/Citratpuf- fer, pH 4,4, 3,2 mmol/l Natriumperborat und 1,9 mmol/l ABTS[¢] (2,2-Acino-bis-(3-ethylbenzathiazolin-6-sulfonsäure)diammoniumsalz) zugegeben und nach einer Stunde Inkubation die Extinktion bei 422 nm bestimmt. Die Ergebnis-se sind aus Fig. 1 und Tabelle II zu ersehen. Sowohl die Wiederfindung als auch die Empfindlichkeit der Bestimmung werden durch die Fraktionierung verbessert.

Tabelle II

Wiederfindung von LH in Seren

| | LH nIU/ml | Wiederfindung Charge | | |
|---|---|---|---|---|
| | | geeignete Referenzcharge | geeignete Fraktionen einer nicht-fraktioniert ungeeigneten Charge (Fraktionen 4 + 5 + 6) | ungeeignete gesamte Charge 13 (ohne Fraktionierung) |
| Kontrollserum 1 | 94,4 | 100 % | 95 % | 111 % |
| Kontrollserum 2 | 13,5 | 100 % | 101 % | 120 % |
| Humanserum 1 | 21,7 | 100 % | 99 % | 118 % |
| Humanserum 2 | 25,5 | 100 % | 100 % | 118 % |
| Humanserum 3 | 2,3 | 100 % | 89 % | 136 % |

**Patentansprüche**

1. Verfahren zur Bestimmung eines Partners in einer Immunreaktion nach dem Immunoassay-Prinzip, wobei einer der Reaktionspartner in fester Phase vorliegt, wobei man als oberflächenaktives Mittel ein Polyethylenoxid-Polypropylenoxid-Blockcopolymer der Formel I

$$HO(CH_2CH_2O)_a(\underset{\overset{|}{CH_3}}{CH}-CH_2O)_b(CH_2CH_2O)_aH$$

einsetzt und worin a einen Wert zwischen 40 und 150 und b einen Wert zwischen 10 und 50 annehmen kann,
**dadurch gekennzeichnet,**
daß man eine hydrophile Blockcopolymer-Zusammensetzung der Formel I verwendet, die in 1%iger wäßriger Lösung (Gew./Vol) eine Oberflächenspannung von mindestens 45 mN/m aufweist und bei der das mittlere molare Verhältnis von Ethylenoxid- zu Propylenoxidgruppen (2a/b) in der Zusammensetzung mindestens 5,8 beträgt.

2. Verfahren nach Anspruch 1,

**dadurch gekennzeichnet,**
daß man eine hydrophile Blockcopolymer-Zusammensetzung der Formel I verwendet, die in 1%iger wäßriger Lösung (Gew./Vol) eine Oberflächenspannung von 45 bis 55 mN/m aufweist und bei der das mittlere molare Verhältnis von Ethylenoxid- zu Propylenoxidgruppen (2a/b) in der Zusammensetzung 5,8 bis 15 beträgt.

**3.** Verfahren nach Anspruch 1 oder 2,
**dadurch gekennzeichnet,**
daß man eine hydrophile Blockcopolymer-Zusammensetzung der Formel I verwendet, bei der die mittlere Zahl der Propylenoxidgruppen pro Propylenoxidblock (b) in der Zusammensetzung nicht größer als 22 ist.

**4.** Verfahren nach einem der Ansprüche 1 bis 3,
**dadurch gekennzeichnet,**
daß man eine hydrophile Blockcopolymer-Zusammensetzung der Formel I verwendet, bei der die mittlere Zahl der Ethylenoxidgruppen pro Ethylenoxidblock (a) in der Zusammensetzung zwischen 50 und 70 ist.

**5.** Verfahren nach einem der vorhergehenden Ansprüche,
**dadurch gekennzeichnet,**
daß man als hydrophile Blockcopolymer-Zusammensetzung eine oder mehrere hydrophile Fraktionen eines kommerziellen Blockcopolymers der Formel I verwendet.

**6.** Verfahren nach Anspruch 5,
**dadurch gekennzeichnet,**
daß man zur Fraktionierung das kommerzielle Blockcopolymer chromatographisch auftrennt.

**7.** Verfahren nach einem der vorhergehenden Ansprüche,
**dadurch gekennzeichnet,**
daß man eine hydrophile Blockcopolymer-Zusammensetzung verwendet, die im wesentlichen frei von Anteilen ist, bei denen das molare Verhältnis von Ethylenoxid- zu Propylenoxidgruppen (2a/b) weniger als 5 beträgt.

**8.** Reagenz zur Durchführung eines Verfahrens nach einem der Ansprüche 1 bis 7, enthaltend als oberflächenaktives Mittel ein Polyethylenoxid-Polypropylenoxid-Blockcopolymer der Formel I

$$HO(CH_2CH_2O)_a(\overset{\displaystyle CH_3}{\overset{|}{C}}H-CH_2O)_b(CH_2CH_2O)_aH$$

**dadurch gekennzeichnet**,
daß es eine hydrophile Blockcopolymer-Zusammensetzung der Formel I enthält, die in einer 1%igen wäßrigen Lösung (Gew./Vol.) eine Oberflächenspannung von mindestens 45 mN/m aufweist und bei der das mittlere molare Verhältnis von Ethylenoxid- zu Propylenoxidgruppen (2a/b) in der Zusammensetzung mindestens 5,8 beträgt.

**9.** Reagenz nach Anspruch 8,
**dadurch gekennzeichnet,**
daß die hydrophile Blockcopolymer-Zusammensetzung der Formel I eine oder mehrere geeignete Fraktionen eines kommerziellen Blockcopolymers der Formel I enthält.

**10.** Reagenz nach Anspruch 8 oder 9 ,
**dadurch gekennzeichnet,**
daß die hydrophile Blockcopolymer-Zusammensetzung nach Formel I weniger als 1 Gew.-% von Anteilen enthält, bei denen das molare Verhältnis von Ethylenoxid- zu Propylenoxidgruppen (2a/b) weniger als 5 beträgt.

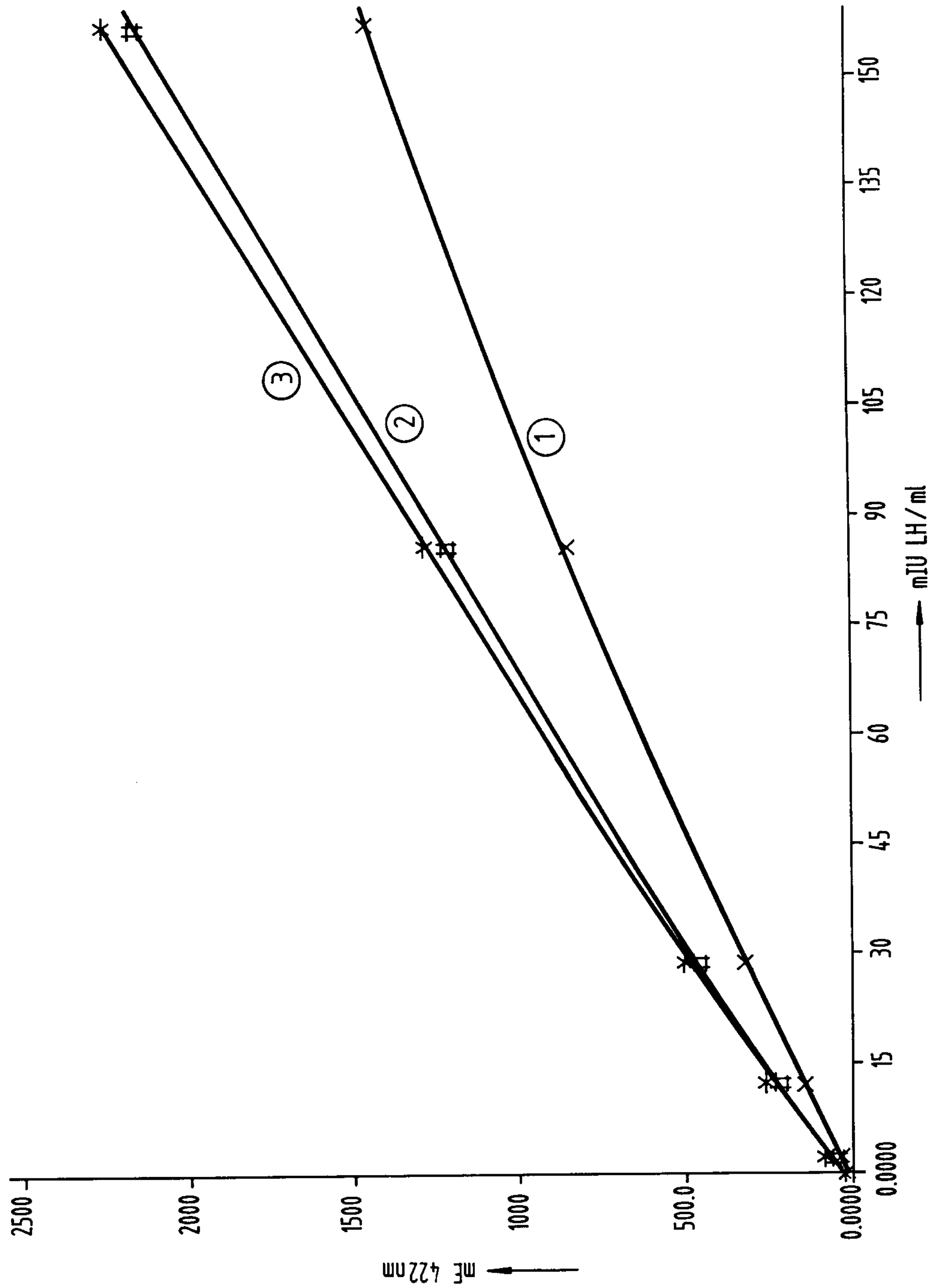
mE 422nm
2500
2000
1500
1000
500.0
0.0000
0.0000
15
30
45
60
75
90
105
120
135
150
mIU LH/ml
1
2
3

Europäisches Patentamt

# EUROPÄISCHER RECHERCHENBERICHT

Nummer der Anmeldung

**EP 91 11 2428**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 215 457 (BOEHRINGER MANNHEIM GmbH)<br>* Insgesamt * | 1-10 | G 01 N 33/53<br>G 01 N 33/543<br>C 08 L 71/02 //<br>G 01 N 33/74 |
| A | EP-A-0 171 946 (TECHNICON INSTRUMENTS CORP.)<br>* Zusammenfassung; Seite 9, Zeile 9 - Seite 10, Zeile 11; Seite 17 * | 1-10 | |
| A | WO-A-9 004 179 (NIPPON OIL AND FATS CO., LTD)<br>* Zusammenfassung; Seite 12 * | 1 | |
| | | | **RECHERCHIERTE SACHGEBIETE (Int. Cl.5)** |
| | | | G 01 N |

Der vorliegende Recherchenbericht wurde für alle Patentansprüche erstellt

| Recherchenort | Abschlußdatum der Recherche | Prüfer |
|---|---|---|
| Den Haag | 30 Oktober 91 | HITCHEN C.E. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument